# EUROPÄISCHE PATENTSCHRIFT

(11) **EP 0 881 023 B1**
(45) Veröffentlichungstag und Bekanntmachung des Hinweises auf die Patenterteilung: **09.11.2005**
(21) Anmeldenummer: 98810492.3
(22) Anmeldetag: 27.05.1998
(51) Int. Cl.: B23D 61/12, B28D 1/12

(54) **Schneid- und Schleifwerkzeug**
Cutting tool with cutting teeth or abrasive cutting edge
Outil de coupe abrasif ou à dents de coupe

(30) Priorität: 28.05.1997 CH 125497; 21.08.1997 CH 195297; 29.10.1997 CH 250297
(43) Veröffentlichungstag der Anmeldung: 02.12.1998
(73) Patentinhaber: maRoc GmbH, 8500 Frauenfeld (CH)
(72) Erfinder: Steiger, Marco, 8524 Uesslingen (CH); Suter, David, 8488 Turbenthal (CH)
(74) Vertreter: Gahlert, Stefan

(56) Entgegenhaltungen:
- US-A- 3 601 114
- US-A- 3 905 105
- US-A- 4 513 742
- US-A- 5 016 356
- US-A- 5 265 340
- US-A- 5 507 763
- US-A- 5 697 835

## Beschreibung

Gegenstand der Erfindung ist ein Schneid- und Schleifwerkzeug mit einer mit einem Werkzeug in Eingriff zu gelangen bestimmten Schneide gemäss Oberbegriff des Patentanspruchs 1 (siehe US-A-5 507 763).

Aus dem Stand der Technik sind Schneidsysteme bekannt, welche Schneidwerkzeuge umfassen, die auf der Abtriebswelle einer Werkzeugmaschine aufgesetzt sind, wobei die Abtriebswelle eine oszillierende Drehbewegung ausführt. Die Drehbewegung umfasst üblicherweise ca. 2 Winkelgrade und eine Frequenz von ca. 20'000/Min. Diese Werkzeuge können für viele Zwecke eingesetzt werden, bzw. sie sind entsprechend ausgebildet. Die meisten dieser bekannten Werkzeuge dienen dazu, um beispielsweise hart verklebte Scheiben an Automobilen ohne Beschädigung von der Karosserie zu lösen, um Karosserieteile zu durchsägen, zu schleifen oder zu schneiden, um Fugen an Fliessen herauszulösen etc. Die Schneiden dieser Werkzeuge sind löffel- oder sichelartig ausgebildet; in jedem Fall verlaufen die in Eingriff mit dem Werkstück gelangenden Schneidenteile bogenförmig, wobei der Bogen mit der durch das Werkzeug ausgeführten bogenförmigen Bewegung übereinstimmt oder geneigt dazu verlaufen kann. So vielfältig die Anwendungsmöglichkeiten dieser Werkzeuge sind; zum Anfertigen seitlich exakt begrenzter und bestimmbarer Einstiche in ein Werkstück sind sie nicht geeignet.

Aufgabe der Erfindung ist es, ein Werkzeug zu schaffen, mit dem Einschnitte und Einstiche in ein Werkstück möglich sind, bei dem die seitlichen Einschnitt- oder Einstichkanten senkrecht zur Oberfläche des Werkstückes liegen und geradlinig verlaufen.

Gelöst wird diese Aufgabe durch ein Schneid- und Schleifwerkzeug gemäss den Merkmalen des Patentanspruchs 1.

Die geradlinige Anordnung der Schneidelemente auf der Schneide sowie der tangentiale Verlauf der Schneide bezüglich des Schwingungsdrehpunktes ermöglicht einen parallel zur Oberfläche des Werkstücks liegenden Schnittverlauf und dadurch die Erzeugung von rechteckigen Ausnehmungen und Einschnitten auch in Ecken von Werkstücken. Das Schneidwerkzeug lässt sich je nach Ausbildung der Schneidelemente für Arbeiten in Holz, Gips, in Verbundwerkstoffen, Stein sowie in Metall verwenden.

In einer vorteilhaften Ausgestaltung der Erfindung liegen die Sägezähne auf zwei in einem stumpfen Winkel zueinander stehenden Geraden. Mit dieser Ausführungsform kann eine im wesentlichen rechtwinklig zum Einstich verlaufende Grundfläche, bzw. eine parallel zur Oberfläche am Stichloch vorgetriebene Schnittfläche erzeugt werden.
Die seitliche Verjüngung des Schneidwerkzeugs und/oder ein zentraler Längsschnitt innerhalb des Scheidwerkzeugs erlauben einen optimalen Spanabtransport und verhindern das Verklemmen des Scheidwerkzeugs zwischen den vom Schneidwerkzeug erzeugten Schnittflächen.
Das Sägewerkzeug weist direkt hinter den Sägezähnen beidseitig eine Verjüngung auf. Vorteilhafterweise ist im zentralen Teil des Sägewerkzeugs ein Längsschnitt ausgebildet, welcher die Späne aufnehmen und nach hinten abführen kann.

Schnitte lassen sich beispielsweise fluchtend zu einer bestehenden Fläche in eine dazu winklig stehende Fläche erzeugen.

Anhand eines illustrierten Ausführungsbeispieles wird die Erfindung näher erläutert. Es zeigen
- Figur 1: eine Seitenansicht einer Werkzeugmaschine (Schneid- und Schleifwerkzeug) mit oszillierender Abtriebswelle,
- Figur 2: einen Grundriss der Werkzeugmaschine in Figur 1,
- Figur 3: eine Aufsicht auf das Schneidwerkzeug und
- Figur 4: einen Längsschnitt längs Linie IV-IV in Figur 3,
- Figur 5: eine Aufsicht auf eine weitere Ausführung eines Schneidwerkzeugs,
- Figur 6: ein weiteres Schneidwerkzeug mit zwei geneigt in einem stumpfen Winkel liegenden Sägezahnreihen.

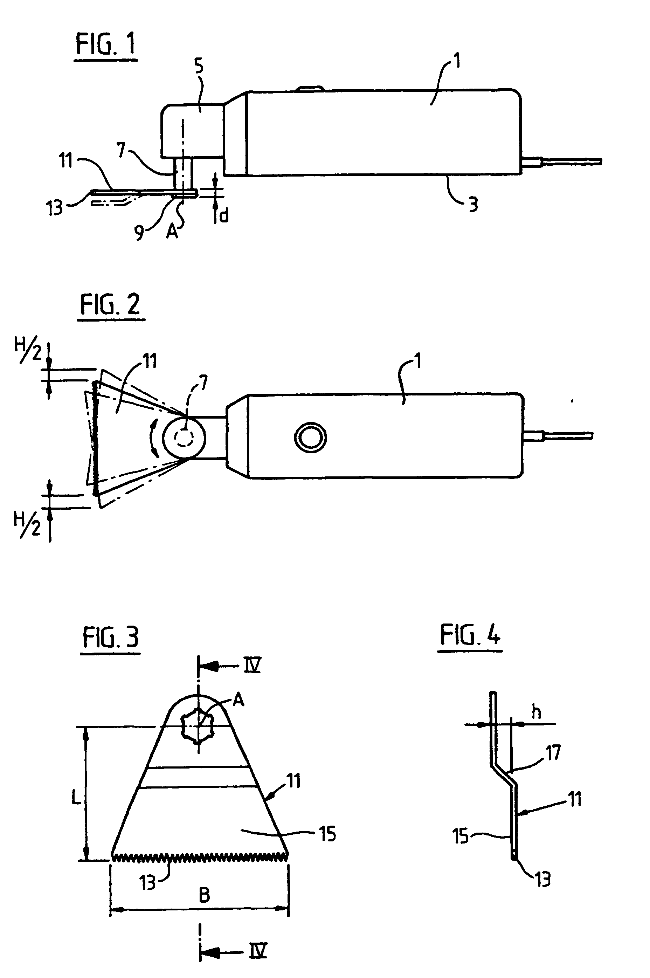

Die in Figur 1 dargestellte motorisch angetriebene und von Hand führbare Werkzeugmaschine 1 weist einen Griffteil 3 mit dem Antriebsmotor und einen Winkelgetriebeteil 5 mit einem darin angeordneten, nicht sichtbaren Winkelgetriebe auf, welches eine nach aussen geführte Abtriebswelle 7 trägt, an deren Ende ein Befestigungsmittel 9, z.B. eine Schraube, aufgesetzt ist, mit welcher ein Werkzeug 11 drehfest mit der Abtriebswelle 7 verbindbar ist. Der Antrieb kann elektrisch oder pneumatisch erfolgen. Die Abtriebswelle 7 vollführt eine oszillierende Drehbewegung um die Achse A in einem Bereich von ca. 2 Winkelgraden und mit einer vorzugsweise stufenlos verstellbaren Frequenz von 0 bis zu 20'000 und mehr Hüben pro Minute.

Das Schneidwerkzeug 11 umfasst eine Schneide 13, welche je nach Einsatzbereich mit einer Zahnung, vorzugsweise mit Kreuzschliff oder geschränktem Schliff, mit Industriediamanten oder für Arbeiten in Metall mit Korund oder anderen abrasiven Stoffen bestückt ist. Das Schneidwerkzeug 11 umfasst im Ausführungsbeispiel eine im wesentlichen trapezförmige Platte 15 an deren Basis die Schneide 13 ausgebildet ist. Vorzugsweise weist die Platte 15 eine Stufe 17 auf, deren Höhe h mindestens der Dicke d des Befestigungsmittels 9 entspricht. Die Länge L des Schneidwerkzeugs, gemessen vom Drehpunkt der Achse A bis zur Schneide 13 kann je nach Einsatzzweck variieren. Zum Erzeugen tiefer Schnitte muss selbstverständlich eine grössere Länge L vorgesehen werden als für das Anbringen von beispielsweise nicht sehr tiefen Nuten. Eine grössere Länge L bewirkt allerdings bei gleichbleibendem Drehwinkel der Werkzeugmaschine 1 einen grösseren Hub H/2. Eine optimale Länge L beträgt etwa 80 mm. Die Breite B der Schneide beträgt im Beispiel ca. 60 mm. Sie kann selbstverständlich für die Erzeugung sehr kleiner Einstiche wesentlich kleiner sein und hängt zudem von der Länge L ab.

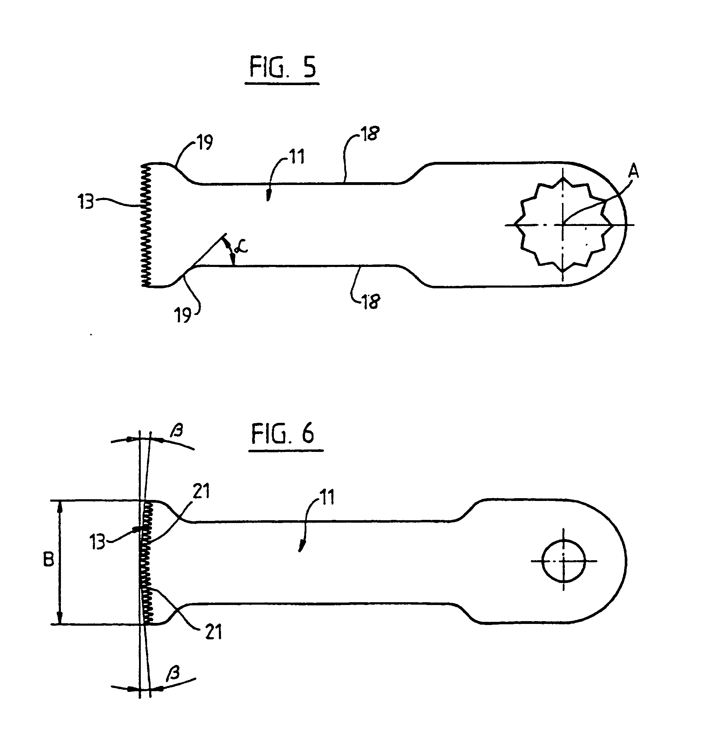

In der Ausgestaltung des Schneidwerkzeugs 11 gemäss Figur 5 verlaufen die Seitenkanten 17 ebenfalls parallel, jedoch liegen sie in geringerem Abstand zueinander. Die Verbindung von der Schneide 13 zu den zurückgesetzten Seitenkanten 18 erfolgt durch Verbindungen 19, die in einem Winkel alpha zu den Seitenkanten 18 verlaufen. Durch die Verschmälerung des Schneidwerkzeugs 11 können die Späne in den verjüngten Bereichen austreten und abgeführt werden.

In einer besonders vorteilhaften Ausgestaltung der Erfindung gemäss Figur 6 liegen die Zahnspitzen der Zähne auf der Schneide 13 nicht auf einer Geraden, sondern die Schneide 13 ist in zwei winklig zueinander verlaufende Abschnitte 21 aufgeteilt. Der Winkel zwischen den wiederum auf Geraden liegenden Zahnspitzen in den Abschnitten 21 ist stumpf. Die Winkel beta der beiden Abschnitte 21 zu einer wie in den Figuren 2 bis 5 beschriebenen Ausführung betragen beispielsweise 1,5° bis 2°. Bei kurzen Schneidwerkzeugen 11 liegt der Winkel beta vorzugsweise über zwei Winkelgraden; bei kürzeren Klingen unter zwei Winkelgraden. Bei einer Klingenlänge von 100 mm, d.h. einem Abstand der Schneide 13 vom Drehpunkt A von 100 mm verwendet man vorzugsweise einen Winkel von 4,6°. Je nach der gewählten Breite B der Schneide 13 kann diese in mehr als zwei Abschnitte 21, die jeweils in einem Winkel beta zueinander verlaufen, aufgeteilt sein.

Die Ausbildung der Schneide 13 hängt, wie bereits oben erwähnt, von der Art des Werkstoffes, der zu schneiden ist, ab. Als sehr vorteilhaft hat sich eine Schneide 13 mit einem Kreuzschliff oder geschränktem Schliff erwiesen für Arbeiten in Holz, Gips, z.B. beim Erzeugen von rechteckigen Einstichen am Bau (in Balken), z.B. Zapfenlöcher, wie sie beim Zusammenfügen von Gebälk benutzt werden, zum Erzeugen von Schattenfugen, zum Heraustrennen von auszutauschenden Fenstern, zum Erzeugen von Schlitzen zum Einschieben einer Platte etc. Bei Arbeiten mit Verbundstoffen aus Kunststoff oder Arbeiten in Stein werden vorzugsweise Industriediamanten in herkömmlicher Weise auf die Schneide 13 aufgebracht. Bei der Bearbeitung von Metall kann beispielsweise Korund als Schneidmaterial eingesetzt werden. Die Verbindung des Schneidmaterials (Korund, Diamanten etc.) mit der Platte 15 erfolgt in bekannter Weise wie bei der Bestückung von anderen Werkzeugen mit den entsprechenden Schneidmaterialien.

Die Handhabung des erfindungsgemässen Schneidwerkzeugs ist sehr einfach. Muss beispielsweise in einem Raum auf einem bestehenden Boden ein Parkettbelag oder ein anderer Belag verlegt werden und soll dieser unter die Türzargen, die bis zum bestehenden Boden reichen, geführt werden, so lässt sich in einfacher Weise ein nur wenige Millimeter über dem bestehenden Boden liegender Einschnitt und, falls die Türzarge in den bestehenden Boden hineinragt, auch bündig zum Boden ein Einschnitt erzeugen, so dass ein sauberer Schlitz zum Einfügen des neuen Bodens entsteht. Der Einstich, der sich mit dem erfindungsgemässen Schneidwerkzeug ausführen lässt, kann nicht nur bündig zu einer Fläche, die parallel zur Schneide liegt, erfolgen, sondern der Einstich kann auch in Ecken von Möbeln oder Räumen parallel zur seitlich verlaufenden Wand erfolgen. Es sind folglich keine Nacharbeiten mit Stechbeitel oder dergleichen notwendig. Das Schneidwerkzeug, insbesondere wenn es sich um ein solches mit Kreuzschliff oder geschränktem Schliff handelt, arbeitet sich im wesentlichen selbstständig, d.h. mit geringstem Druck, in das zu schneidende Material hinein. Die geradlinig verlaufende Schneide bewirkt denn auch, dass der Grund des erzeugten Schlitzes oder Loches bis in die Ecken gleich tief verläuft. Dies im Gegensatz zu bogenförmigen Werkzeugen, mit denen die seitlichen Bereiche immer weniger tief sind und zudem eine nicht entsprechend gleich saubere Schnittkante aufweisen.

## Patentansprüche

1. Schneid- und Schleifwerkzeug mit einer mit einem Werkstück im Eingriff zu gelangen bestimmten Schneide für eine motorisch angetriebene, handgeführte Werkzeugmaschine, umfassend eine oszillierende Drehbewegung ausführende Werkzeugabtriebswelle zum Aufsetzen und Befestigen des Schneidwerkzeugs, dessen mit Zähnen versehene Schneide (13) in einem Abstand (L) zur Achse (A) des Schneidwerkzeugs (11) angeordnet ist, wobei die Spitzen der Zähne an der Schneide (13) entweder auf einer Geraden oder auf zwei in stumpfem Winkel zueinander liegenden geraden Abschnitten (21) liegen, **dadurch gekennzeichnet, dass** die Schneide (13) an einer Platte (15) angebracht ist, die auschließenden die Schneide einen Bereich aufweist, der schmaler ist als die Schneide (13) und keine Zähne aufweist.

2. Schneid- und Schleifwergzeug nach Anspruch 1, **dadurch gekennzeichnet, dass** die Spitzen der Sägezähne eines Abschnitts (21) in einem Winkel (beta) von 1,5° bis 4,6°, vorzugsweise 1,5° bis 2° zu den Spitzen der Sägezähne des benachbarten Abschnitts (21) liegen.

3. Schneid- und Schleifwerkzeug nach einem der Ansprüche 1 oder 2, **dadurch gekennzeichnet, dass** die Platte (15) eine Stufe (17) aufweist, deren Höhe (h) gleich oder grösser ist als die Dicke (d) des Befestigungsmittels (9) an der Abtriebswelle (7) der Werkzeugmaschine (1).

4. Schneid- und Schleifwerkzeug nach einem der Anspruche 1 bis 3, **dadurch gekennzeichnet, dass** in der Platte (15) seitliche Rücksprünge als Kanäle zur Spanabfuhr ausgebildet sind.

## Claims

1. Cutting and grinding tool with a cutting edge designed to engage with a workpiece for a motor-driven, hand-held machine tool comprising a tool output shaft performing an oscillating rotary motion for positioning and fastening the cutting tool whereby the cutting edge (13) fitted with teeth is arranged at a distance (L) to the axis (A) of the cutting tool (11), whereby the tips of the teeth on the cutting edge (13) lie either on one straight section or on two straight sections (21) arranged at an obtuse angle to each other, **characterised in that** the cutting edge (13) is attached to a plate (15), which has an area adjoining the cutting edge that is narrower than the cutting edge (13) and has no teeth.

2. Cutting and grinding tool according to claim 1, **characterised in that** the tips of the sawteeth in one section (21) lie at an angle (beta) of 1.5° to 4.6°, preferably 1.5° to 2° to the tips of the sawteeth in the adjacent section (21).

3. Cutting and grinding tool according to either claim 1 or claim 2, **characterised in that** the plate (15) has a step (17) the height of which (h) is equal to or greater than the thickness (d) of the fastening means (9) on the output shaft (7) of the machine tool (1).

4. Cutting and grinding tool according to any one of claims 1 to 3, **characterised in that** the plate (15) contains lateral recesses as channels for chip removal.

## Revendications

1. Outil de coupe et de meulage comportant une lame de coupe, destinée à entrer en prise avec une pièce à usiner, pour une machine outil, qui est actionnée par un moteur et guidée à la main et qui comporte un arbre de sortie effectuant un mouvement de rotation oscillant et destiné au montage et à la fixation de l'outil de coupe, dont la lame de coupe (13), munie de dents, est disposée à une distance (L) de l'axe (A) de l'outil de coupe (11), les sommets des dents sur la lame de coupe (13) étant situés soit une section droite, soit sur deux sections droites (21) formant un angle obtus entre elles, **caractérisé en ce que** la lame de coupe (13) est agencée sur une plaquette (15) qui comporte une zone adjacente à la lame de coupe (13) qui est plus étroite que la lame de coupe (13) et ne comporte pas de dents.

2. Outil de coupe et de meulage selon la revendication 1, **caractérisé en ce que** les sommets des dents de scie d'une section (21) forment avec les sommets des dents de scie de la section (21) adjacente un angle (bêta) de 1,5° à 4,6°, de préférence 1,5° à 2°.

3. Outil de coupe et de meulage selon la revendication 1 ou 2, **caractérisé en ce que** la plaquette (15) comporte un décrochement (17) dont la hauteur (h) est égale ou supérieure à l'épaisseur (d) du moyen de fixation (9) sur l'arbre de sortie (7) de la machine-outil (1).

4. Outil de coupe et de meulage selon l'une quelconque des revendications 1 à 3, **caractérisé en ce que** des épaulements latéraux sont réalisés sous forme de conduits dans la plaquette (15) pour l'évacuation des copeaux.
